# EUROPEAN PATENT APPLICATION

(11) **EP 1 627 597 A1**
(43) Date of publication of application: **22.02.2006**
(21) Application number: 05255056.3
(22) Date of filing: 16.08.2005
(51) Int. Cl.: A61B 5/0408, A61G 11/00

(54) **Device for detecting bioelectric signals using capacitive electrodes**

(30) Priority: 17.08.2004 US 919461; 16.06.2005 US 153620
(71) Applicant: Quantum Applied Science and Research, Inc., San Diego, CA 92121 (US)
(72) Inventor: Fridman, Igor, San Diego California 92103 (US); Hervieux, Paul, San Diego California 92126 (US); Matthews , Robert, San Diego California 92126 (US)
(74) Representative: Jeffrey, Philip Michael

(57) **Abstract**

A system for unobtrusively measuring bioelectric signals developed by an individual includes multiple sensors, one or more of which constitutes a capacitive sensor, embedded into or otherwise integrated into an object, such as a chair, bed or the like, used to support the individual. In one preferred embodiment, multiple capacitive sensors are incorporated into a pad provided in an incubator for unobtrusively measuring bioelectric signals from a baby under supervised care. In any case, the object serves as mounting structure that holds the sensors in place. The sensors are preferably arranged in the form of an array, with particular ones of the sensors being selectable from the array for measuring the bioelectric signals which are transmitted, such as through a wireless link, for display and/or analysis purposes.

## Description

### CROSS-REFERENCE TO RELATED APPLICATIONS

The present invention represents a continuation-in-part of U.S. Patent Application Serial No. 10/919,461 filed August 17, 2004.

### BACKGROUND OF THE INVENTION

### 1. Field of the Invention

The present invention pertains to the art of measuring bioelectric signals and, more particularly, to a system for unobtrusively measuring bioelectric signals developed by an individual.

### 2. Discussion of the Prior Art

It is widely known that electric fields are developed in free space from many different sources. For example, organs in the human body, including the heart and brain, produce electric fields. For a variety of reasons, it is often desirable to measure these electric fields, such as in performing an electrocardiogram (ECG). Actually, the measuring of bioelectric signals can provide critical information about the physiological status and health of an individual, and are widely used in monitoring, evaluating, diagnosing and caring for patients. Basically, prior methods of measuring electric potentials associated with a human employ securing gel-coated electrodes directly to the skin of a patient. Obviously, this requires preparation and application time, while being quite discomforting to the patient.

More specifically, resistive electrodes have been predominantly employed in connection with measuring electric potentials produced by animals and human beings. As the resistive electrodes must directly touch the skin, preparation of the skin to achieve an adequate resistive connection is required. Such resistive electrodes are the standard for current medical diagnostics and monitoring, but the need for skin preparation and contact rule out expanding their uses. Although attempts have been made to construct new types of resistive electrodes, such as making an electrically conductive fabric, providing a miniature grid of micro-needles that penetrate the skin, and developing chest belt configurations for heart related measurements or elasticized nets with resistive sensors making contact via a conductive fluid for head related measurements, these alternative forms do not overcome the fundamental limitation of needing to directly contact the skin. This limitation leads to an additional concern regarding the inability to maintain the necessary electrical contact based on differing physical attributes of the patient, e.g. amount of surface hair, skin properties, etc.

Another type of sensor that can be used in measuring biopotentials is a capacitive sensor. Early capacitive sensors required a high mutual capacitance to the body, thereby requiring the sensor to also touch the skin of the patient. The electrodes associated with these types of sensors are strongly affected by lift-off from the skin, particularly since the capacitive sensors were not used with conducting gels. As a result, capacitive sensors have not been found to provide any meaningful benefits and were not generally adopted over resistive sensors. However, advances in electronic amplifiers and new circuit techniques have made possible a new class of capacitive sensor that can measure electrical potentials when coupling to a source in the order of 1 pF or less. This capability makes possible the measurement of bioelectric signals with electrodes that do not need a high capacitance to the subject, thereby enabling the electrodes to be used without being in intimate contact with the subject.

To enhance the measurement of bioelectric signals, there still exists a need for a system which can unobtrusively measure the signals with minimal set-up or preparation time. In addition, there exists a need for a bioelectric signal measuring system which is convenient to use, both for the patient and an operator, such as a nurse, doctor or technician. Furthermore, there exists a need for an effective bioelectric signal measuring system which can be used on a patient without the patient being cognitive of the system so as to require an absolute minimum intervention or assistance by the patient, particularly in situations wherein the patient cannot aid a nurse, doctor or the like, such as in the case of an infant or an unconscious individual. Specifically, a truly unobtrusive measurement system which does not require patient preparation is needed.

### SUMMARY OF THE INVENTION

The present invention is directed to a system for unobtrusively measuring bioelectric signals developed by an individual, inclusive of a human or animal. The measurement system enables bioelectric signals to be collected through multiple sensors, one or more of which constitutes a capacitive-type sensor, carried by an object against which the individual is positioned. In this manner, the object serves as mounting structure that holds the sensors in place relative to both each other and the individual to assure proper system operation. The sensors are preferably not in direct contact with the skin of the user, but rather are spaced from the user by a layer of material, such as a biocompatible and non-conductive material, e.g. cushioning foam or the like.

In accordance with one embodiment of the invention, the sensor system is formed or otherwise integrated into a pad that can be laid over a chair, stretcher, gurney or bed, including an incubator. The pad can be readily arranged in a compact configuration for transporting the same. For instance, the pad can be rolled-up into a compact and convenient form for transporting or storing purposes. In the alternative, the sensor system could be embedded directly in a backrest of the chair, beneath a layer of the stretcher or gurney, or in the foam or fabric associated with the bed. With this arrangement, an individual need only sit in the chair or simply lay on any one of the stretcher, gurney or bed in order for the desired electric signals to be sensed.

Regardless of the particular implementation, the sensor system of the invention is integrated into an object against which an individual rests in a normal manner such as he/she would do when usually encountering the object, to enable bioelectric signals to be continuously measured in an extremely convenient, unobtrusive and effective way, with little or no intervention needed on the part of the individual. Additional objects, features and advantages of the present invention will become more readily apparent from the following detailed description of preferred embodiments when taken in conjunction with the drawings wherein like reference numerals refer to corresponding parts in the several views.

### BRIEF DESCRIPTION OF THE DRAWINGS

Figure 1 schematically illustrates the basic sensor system arrangement of the invention;
Figure 2A is a perspective view illustrating the incorporation system of the invention directly into a mat or pad;
Figure 2B depicts the pad of Figure 2A in a compact, transporting or storing configuration;
Figure 3 is a perspective view illustrating the use of the sensor system of the invention in combination with a chair;
Figure 4 is a perspective view illustrating the use of the sensor system of the invention in combination with a stretcher or gumey;
Figure 5 is a perspective view illustrating the use of the sensor system of the invention in combination with a bed; and
Figure 6 is a perspective view of an incubator incorporating the sensor system of the invention.

### DETAILED DESCRIPTION OF THE PREFERRED EMBODIMENTS

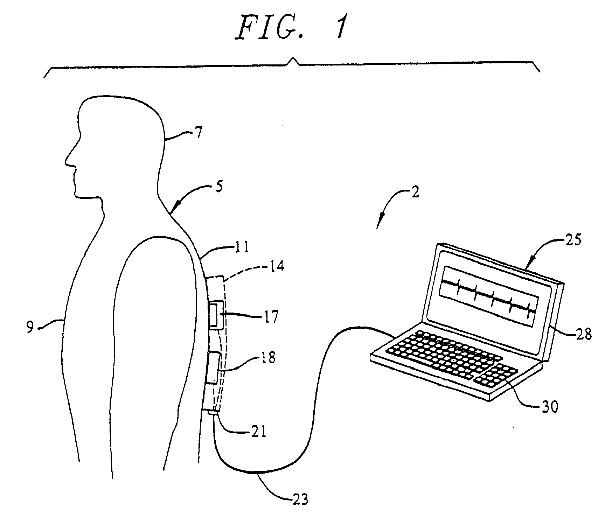

With initial reference to Figure 1, a sensor system constructed in accordance with the present invention is generally indicated at 2. In general, sensor system 2 functions to measure biopotentials of an individual 5, such as a medical patient, animal, test subject or the like. As shown, individual 5 includes a head 7, a chest 9 and back 11, with back 11 being positioned against an object which forms part of sensor system 2. In the embodiment shown, the object constitutes a pad 14. More specifically, sensor system 2 includes pad 14 having embedded or otherwise integrated therein at least first and second sensors 17 and 18. In accordance with the invention, at least first sensor 17 constitutes a capacitive-type sensor and, in the most preferred embodiment of the invention, both first and second sensors 17 and 18 constitute capacitive-type sensors.

As shown, each of first and second sensors 17 and 18 is preferably hardwired to a connector 21 and linked through a cable 23 to a remote control unit 25 of sensor system 2. In the embodiment shown, control unit 25 constitutes a laptop computer having a display panel 28 and a keyboard 30. As will be detailed more fully below, the use of sensor system 2 enables individual 5 to be supported against pad 14 whereby a bioelectric field produced by individual 5 can be sensed through first and second sensors 17 and 18, with bioelectric signals being transmitted to control unit 25 for analysis and display purposes. That is, individual 5 will inherently produce time-varying potentials which will be sensed through first and second sensors 17 and 18. As first and second sensors 17 and 18 preferably constitute capacitive-type sensors, no electrically conducting path to individual 5 is needed. In other words, no flow of real current (electrons) occur between individual 5 and first and second sensors 17 and 18 such that first and second sensors 17 and 18 need not be in physical contact with individual 5. Therefore, the use of capacitive-type sensors enables first and second sensors to be embedded or otherwise integrated into an object against which individual 5 is positioned. Various particular embodiments of the invention will be set forth below but, at this point, it should simply be noted that sensor system 2 can be employed to measure the bioelectric field associated with individual 5 by simply supporting individual 5 against pad 14. In this manner, an extremely unobtrusive and convenient sensing system is established which requires no specific set-up or intervention.

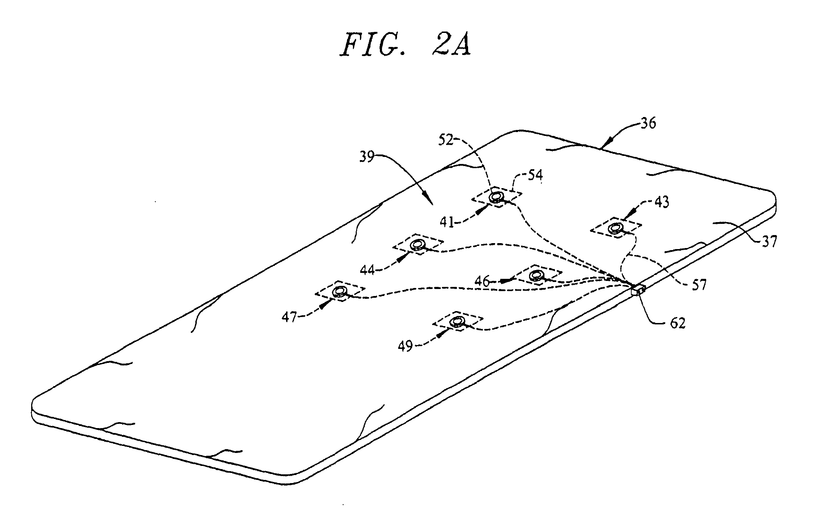

Reference will now be made to Figure 2A which depicts a particular embodiment of the invention. In accordance with this embodiment, sensor system 2 is incorporated into a pad or mat 36 including a cushion layer 37 which is preferably constituted by foam or another biocompatible and non-conductive material. Embedded within pad 36 is a sensor array 39 which is shown to include a plurality of sensors 41-49. As shown, sensors 41-49 are arranged in various row and columns. However, sensors 41-49 can actually be more randomly arranged or repositionable relative to pad 36. In any case, each sensor 41-49 preferably constitutes a capacitive-type sensor and includes a capacitive-type electrode 52 having an associated mounting strip 54. Each electrode 52 is linked through one or more conductors 57 to a connector 62 that is exposed from pad 36. Connector 62 is adapted to be interconnected to a control unit 25.

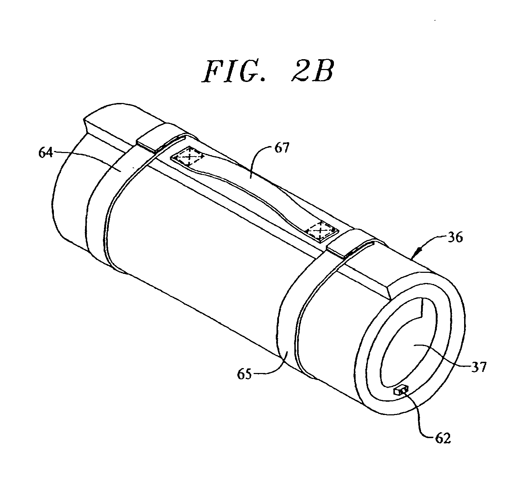

To increase the versatility and portability of the invention, pad 36 can preferably be placed in a compact and convenient configuration for transporting and/or storing purposes. Figure 2B illustrates pad 36 has been rolled-up and retained in this configuration by means of integral straps 64 and 65. In accordance with the invention, straps 64 and 65 can be selectively secured or released by means of various types of fasteners known in the art, including snap-type fasteners, VELCRO, buttons, buckles, clasps or the like (not shown). In addition, pad 36 preferably incorporates an integral handle 67 to further enhance the portability thereof.

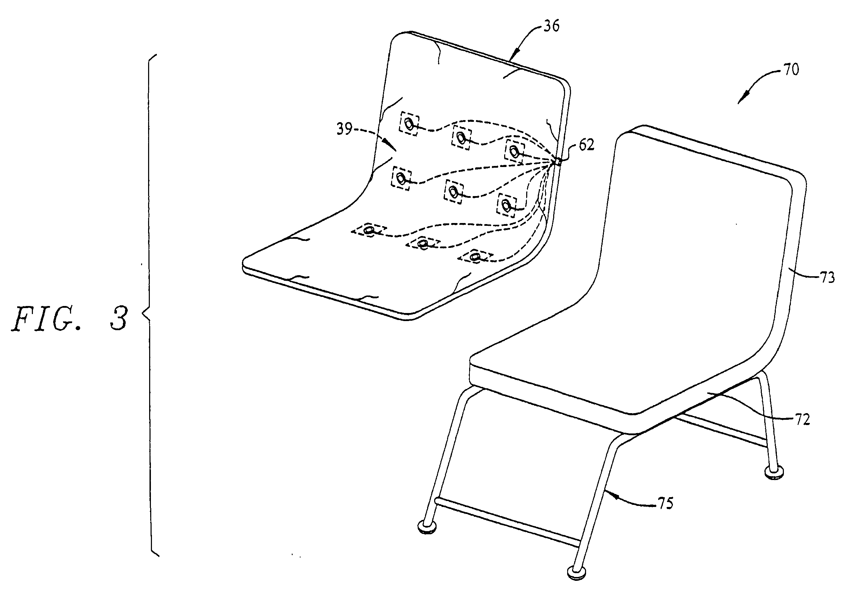

Pad 36 can take various shapes and forms in accordance with the invention, including that of pad 14. Figure 3 illustrates an arrangement wherein pad 14 conforms to, and is adapted to be supported upon, a chair 70. As shown, chair 70 includes a seat portion 72, a back portion 73 and a supporting frame 75. Once pad 36 is laid upon both seat portion 72 and back portion 73 and interconnected to control unit 25, individual 5 need only sit in chair 70 in order for sensor system 2 to be able to sense the bioelectric field developed by individual 5. As individual 5 does not need to be prepped, such as by having electrodes directly attached to back 11, individual 5 is not at all inconvenienced and, in fact, may not even be aware that the bioelectric field is being sensed. Through the use of capacitive-type sensors 41-49, the bioelectric signals can even be advantageously sensed through clothing worn by individual 5, as well as cushion 37 of pad 36.

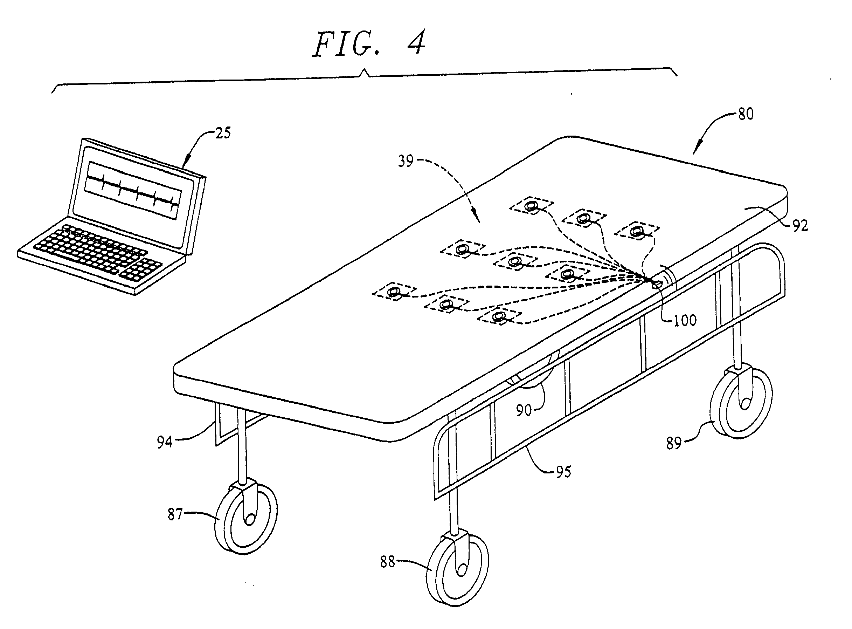

Based on the above, it should be readily apparent that sensor system 2 of the present invention can be incorporated into various objects against which an individual 5, who produces a bioelectric field to be measured, is adapted to be supported. Although sensor array 39 is shown in Figure 3 to be incorporated into pad 36 that is placed upon chair 70, it should be realized that other arrangements are possible in accordance with the invention, such as having sensor array 39 directly integrated into seat and/or back portions 72 and 73 of chair 70. Figure 4 illustrates another embodiment of the invention wherein sensor system 2 is incorporated into a stretcher or gurney 80 which is supported by casters 87-90 for mobility purposes. In any case, gurney 80 includes a table portion 92, as well as side protectors or rails 94 and 95. As shown, sensor array 39 is embedded within table portion 92. With this arrangement, individual 5 need merely lay, either on chest 9 or back 11, on table portion 92 in order for measuring of the bioelectric field.

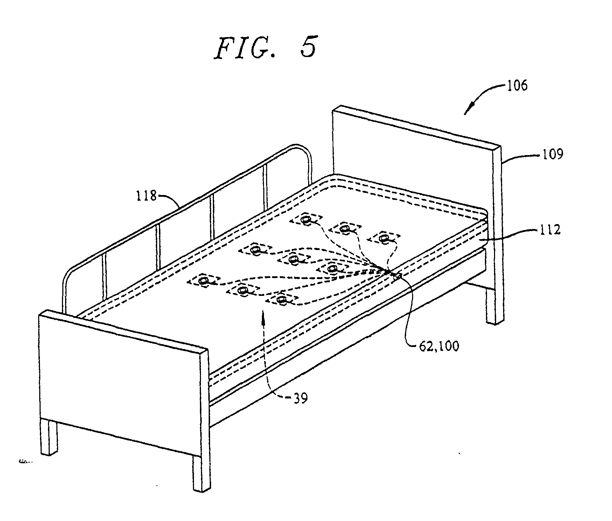

Figure 4 also illustrates another aspect of the present invention. In the embodiment set forth above, sensor array 39 was linked to control unit 25 through a cable 23. In this embodiment, the plurality of sensors 41-49 are linked through a connector 100 which constitutes a wireless transmitter. With this arrangement, RF, infrared or the like type signals can be employed to communicate sensor array 39 to control unit 25. Figure 5 illustrates a still further embodiment of the invention wherein sensor system 2 is incorporated into a bed 106 having a frame 109 and a mattress 112. More specifically, sensor array 39 is embedded into mattress 112 or other cushion which has exposed therefrom an associated connector 21, 100.

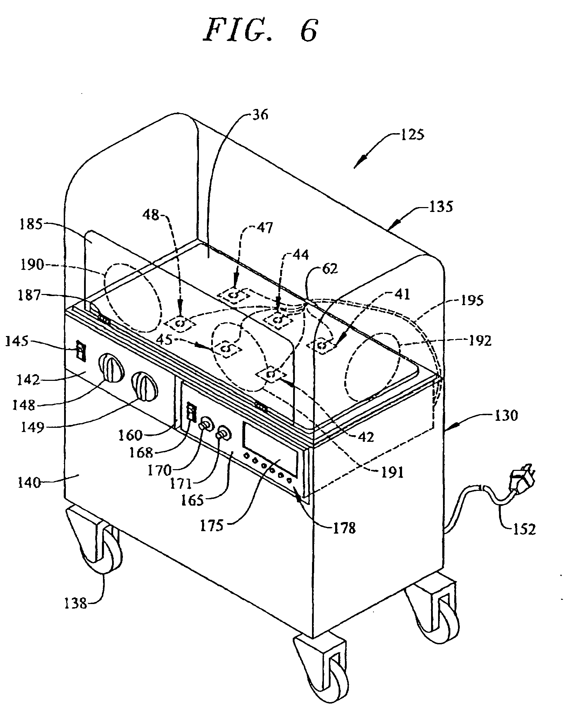

Figure 6 illustrates a particularly preferred form of the invention wherein a small sized pad 36 has been incorporated in an incubator 125. As shown, incubator 125 includes a base 130 upon which is seated an upper transparent housing 135. As illustrated, base 130 is preferably mounted upon a plurality of casters, one of which is indicated at 138, to enhance the mobility of incubator 125. Base 130 includes a frontal portion 140 which defines a control panel 142. In the embodiment illustrated, control panel 142 includes a power button 145 and control knobs 148 and 149. Power button 145 is linked to a power cord 152 for use in supplying power to incubator 125. Control knobs 148 and 149 are utilized in connection establishing operating parameters for incubator 125, including temperature and air quality parameters within housing 135.

Frontal portion 140 of base 130 is also preferably provided with a recess 160 within which is positioned a controller 165 for sensor system 2. In the embodiment shown, controller 165 includes a power button 168, adjustment knobs 170 and 171, a display screen 175 and a row of sensor select buttons 178. Preferably, row 178 has a number of buttons corresponding to the number of sensors in mat 36. In the embodiment shown, row 178 includes six buttons corresponding to sensors 41, 42, 44, 45, 47 and 48. With this arrangement, a doctor, nurse or technician can, if desired, manually select the particular ones of sensors 41, 42, 44, 45, 47 and 48 to be utilized in connection with measuring the bioelectric signals from an infant placed upon mat 36 in housing 135.

As also shown in this figure, housing 135 includes a front panel 185 that is hinged at 187 to the remainder of panel 185. Panel 185 can be shifted to provide unencumbered access to within housing 135. On the other hand, in a manner known in the art of incubators, various portals 190-192 can be provided within housing 135. Although not shown for the sake of clarity of the drawings, each portal 190-192 would include sleeve-type structure enabling a nurse, doctor or the like to place protected hands within housing 135 in order to interact with an infant in incubator 125. In any case, it is shown that the plurality of sensors 41, 42, 44, 45, 47 and 48 are linked through connector 62 and a cable 195 to send biosignals to controller 165 in accordance with the invention.

It should be noted that this embodiment of the present invention is considered to be particularly advantageous as infants needing to be housed in incubator 125 require special care and attention. Sensing various bioelectric signals from an infant placed in incubator 125 in accordance with the present invention can be advantageously done through clothing worn by the infant, as well as the cushion of pad 36. The number of sensors incorporated in pad 36 and the activation/de-activation of each sensor can be readily established. If this degree of monitoring of an infant is not required, pad 36 and controller 165 can be easily removed and transported to another location, with pad 36 being arranged in a compact configuration as discussed above with reference to Figure 2B.

As indicated above, sensor system 2 of the present invention constitutes an unobtrusive measurement system for bioelectric fields. To this end, sensor array 39 is naturally brought into adequate physical proximity to individual 5 by merely positioning a respective body portion of individual 5 against the object, whether it be pad 14, pad 36, chair 70, gurney 80, bed 106, a crib, an incubator, a couch, a wall or the like. To this point, sensor system 2 has been disclosed for use in sensing electric fields produced by a heart of individual 5. However, sensor system 2 of the invention can be employed to measure electric fields produced by other organs of individual 5, such as the brain. In this case, head 7 of individual 5 would be positioned against and supported by an object, such as a cushioned headrest, provided as part of a scanning device into which sensor array 39 is integrated. In any case, sensor system 2 does not require attention from individual 5 for proper operation. For instance, individual 5 need not grip a particular grounding element, apply conducting fluids or the like in order for the electric field to be measured. The object itself serves as the mounting structure that holds the plurality of sensors 41-49 in place relative to individual 5 and to each other. Again, capacitive-type sensors are preferably employed to avoid the need for direct contact with the skin of individual 5 by electrodes 52. In general, capacitive-type sensors 41-49 are able to measure biopotentials with total input capacitance less than approximately 50 pF and preferably less than 1 pF. For each of the chair, gurney and bed embodiments, it is preferred to stack or run averages of multiple sensed wave forms in order to provide a clinical quality electrocardiogram (ECG). Although sensor array 39 is preferably utilized, it is only necessary that two or more sensors be located in the region where the biopotential signal is to be measured. Sensor array 39 is preferably employed in order to enable a select set of sensors 41-49 to be utilized for any given measurement. More specifically, a nurse, doctor, technician or the like can activate select ones of sensors 41-49 through control unit 25 for any given procedure, or a software algorithm can be used to automatically make the selection based on established criteria.

Although described with reference to preferred embodiments of the invention, it should be readily understood that various changes and/or modifications can be made to the invention without departing from the spirit thereof. Regardless of the particular implementation, the sensor system of the invention is integrated into an object against which an individual rests to enable bioelectric signals to be continuously measured in an extremely convenient, unobtrusive and effective manner, with little or no intervention needed on the part of the individual producing the bioelectric field being measured. In the overall system, the bioelectric signals can be pre-processed, either prior to or by the remote control unit. For instance, the difference between the outputs of one or more sensors can be taken before transmitting the data or simply prior to further analyzing the data. In any event, the invention is only intended to be limited by the scope of the following claims.

## Claims

1. An incubator comprising:
a base;
an upper housing arranged on the base;
a pad arranged in the housing and upon which an infant, who produces a bioelectric field to be measured, is adapted to be supported;
a plurality of electrical sensors integrated into the pad as an array, at least one of the plurality of electrical sensors being constituted by a capacitive-type electrical sensor; and
a controller located outside the housing and electrically linked to each of the plurality of electrical sensors, wherein activation of one or more of the plurality of electrical sensors enables the bioelectric field to be unobtrusively measured.

2. The incubator according to claim 1, wherein the plurality of electrical sensors are embedded into the pad.

3. The incubator according to claim 1, wherein the pad defines a mattress for the incubator.

4. The incubator according to claim 1, wherein the controller receives, processes, stores and displays the bioelectric signals.

5. The incubator according to claim 1, wherein the controller includes means for choosing select ones of the plurality of electrical sensors in the array to sense the bioelectric field.

6. The incubator according to claim 5, wherein each of the plurality of electrical sensors constitutes a capacitve-type electrical sensor.

7. The incubator according to claim 1, wherein the pad is removable from the housing for transporting separate from the housing.

8. The incubator according to claim 7, wherein the pad includes a handle for carrying the pad.

9. A method of sensing bioelectric signals from an infant producing a bioelectric field in an incubator having a housing including an upper portion comprising:
supporting a body portion of the infant upon a pad having a plurality of integrated electrical sensors within the housing, with at least one of the plurality of electrical sensors being constituted by a capacitive-type sensor;
measuring bioelectric signals with the plurality of electrical sensors; and
transmitting the bioelectric signals associated with the bioelectric field to a controller.

10. The method of claim 9, wherein the controller is carried by the incubator such that the controller is transported with the housing.

11. The method of claim 9, further comprising: selecting ones of the plurality of electrical sensors from an array of the plurality of electrical sensors.

12. The method of claim 9, further comprising: receiving, processing, storing and displaying the bioelectric signals.

13. The method of claim 9, further comprising:
removing the pad from the housing;
placing the pad in a compact configuration; and
transporting the pad separate from the housing.

14. The method of claim 13, wherein placing the pad in the compact configuration constitutes rolling up the pad, along with the plurality of electrical sensors.

15. The method of claim 13, further comprising: using a handle attached to the pad in transporting the pad.
